# EUROPEAN PATENT SPECIFICATION

(11) **EP 4 486 413 B1**
(45) Date of publication and mention of the grant of the patent: **11.11.2026**
(21) Application number: 22843258.9
(22) Date of filing: 21.12.2022
(51) Int. Cl.: A61M 5/145, A61M 5/142

(54) **INFUSION DEVICE HAVING A DRIVE MECHANISM FOR DRIVING A PUSHER DEVICE**
INFUSIONSVORRICHTUNG MIT ANTRIEBSMECHANISMUS ZUM ANTRIEB EINER VORSCHUBEINRICHTUNG
DISPOSITIF DE PERFUSION AYANT UN MÉCANISME D'ENTRAÎNEMENT POUR ENTRAÎNER UN DISPOSITIF POUSSOIR

(30) Priority: 04.03.2022 EP 22315012
(43) Date of publication of application: 08.01.2025
(73) Proprietor: Fresenius Vial SAS, 38590 Brézins (FR)
(72) Inventor: ARCHAT, Damien, 38000 Grenoble (FR); LOUSSERT, Guillaume, 38980 Virville (FR)
(74) Representative: Fresenius Kabi Deutschland GmbH
(86) International application number: PCT/EP2022/087153
(87) International publication number: WO 2023/134986

(56) References cited:
- EP-A2- 0 314 880
- WO-A1-2018/046313
- WO-A1-2020/160821
- DE-A1- 19 804 354

## Description

The invention relates to an infusion device for administering a medical fluid to a patient according to the preamble of claim 1.

An infusion device of this kind comprises a housing having a receptacle for receiving a syringe. A pusher device is movable along a pushing direction for acting onto a piston of a syringe received on the receptacle. A drive mechanism is operative to drive the pusher device, the drive mechanism comprising a first drive element, a second drive element being in threaded engagement with the first drive element, and an electric drive for rotating the first drive element. The second drive element is translationally movable with respect to the first drive element by rotating the first drive element.

Within an infusion device in the shape of a syringe pump, as it for example is known from US 2012/0215170 A1, WO2018046313A1 or WO 2020/249647 A1, a pusher device serves to act onto a piston of a syringe in order to push, during operation, the piston of the syringe into a syringe tube in order to deliver a medical fluid from the syringe towards a patient in the context of an ongoing infusion operation. The pusher device herein is driven by a drive mechanism comprising a driving rod carrying a screw thread. Clutch elements are connected to the pusher device, the clutch elements being shaped as half nuts and engaging in a clutched state with the screw thread of the driving rod such that a rotation of the driving rod causes a translational movement of the pusher device. The assembly of the clutch elements in addition is operatively connected to a braking device which serves to brake the pusher device such that the pusher device is held in position when the brake device is actuated. In DE 198 04 354 A1 another syringe pump is disclosed.

In an infusion device there is a general desire to simplify the construction and to ease the drive mechanism in order to reduce the number of movable components. Herein, operation shall be reliable in that an operative connection between the electric drive and the pusher device shall be reliably established in order to transfer forces for moving the pusher device during an infusion operation. An undesired disruption of a force transfer shall be avoided. It furthermore is desirous to provide a drive mechanism having a good efficiency and exhibiting a low wear and tear.

It is an object of the instant invention to provide an infusion device which allows a simplified construction while offering a good operational reliability and efficiency.

This object is achieved by means of an infusion device comprising the features of claim 1.

Accordingly, one of the first drive element and the second drive element is a spindle having an outer threading and the other of the first drive element and the second drive element is a spindle nut being in threaded engagement with the outer threading of the spindle. The drive mechanism comprises a brake mechanism for rotationally fixing, in a brake state, the second drive element such that a rotation of the first drive element causes a translational movement of the second drive element, and for rotationally releasing, in a release state, the second drive element to allow a rotation of the second drive element with respect to the first drive element while the first drive element is at standstill.

Within the infusion device, the drive mechanism is designed as a spindle drive having a first drive element and a second drive element, one of which is a spindle and the other of which is a spindle nut. By causing a rotation of the spindle and the spindle nut with respect to each other, the spindle and the spindle nut may be translationally moved with respect to each other in order to translationally move the pusher device with respect to the housing of the infusion device. The spindle nut herein is in threaded engagement with the spindle, such that a rotation of one of the drive elements causes a translational movement of the other drive element. As the spindle nut non-releasably is arranged on the spindle and engages, via an inner threading, with the outer threading of the spindle, a reliable force transfer and movement may be achieved.

Generally, it is not relevant whether the spindle or the spindle nut is in operative connection with the electric drive and is rotated by the electric drive. Rather, different options exist.

In a first option, the spindle may be rotatable and may be in operative connection with the electric drive, such that the spindle may be driven by the electric drive for causing a translational movement of the pusher device. In this case the first drive element is formed by the spindle.

In another, second option, the spindle nut may be rotatable and may be in operative connection with the electric drive, such that the spindle nut may be driven by the electric drive for causing a translational movement of the pusher device. In this case the first drive element is formed by the spindle nut.

The first drive element herein may be arranged on the housing of the infusion device or on the pusher device. In the first case the electric drive is arranged on the housing of the infusion device. In the second case the electric drive is arranged on the pusher device.

By driving the first drive element a moving force may be produced by the electric drive and may be introduced in the pusher device in order to move the pusher device along the pushing direction with respect to the housing of the infusion device. When driving the first drive element the second drive element shall be held fixed (on the functional component that the second drive element is associated with, i.e., the pusher device or the housing of the infusion device) such that a rotation of the first drive element as driven by the electric drive may be translated into a translational movement of the second drive element with respect to the first drive element in order to longitudinally move the pusher device with respect to the housing of the infusion device along the pushing direction. The second drive element may be held rotationally fixed with respect to the housing of the infusion device (in case the first drive element is rotatably arranged on the pusher device and is driven by the electric drive on the pusher device) or with respect to the pusher device (in case the first drive element is rotatably arranged on the housing of the infusion device and is driven by the electric drive on the housing of the infusion device).

The infusion device shall allow a manual movement of the pusher device in order to establish a connection between the pusher device and a syringe received on the receptacle of the housing. Hence, the operative connection of the pusher device to the drive mechanism shall be releasable, such that in a release state the pusher device is movable with respect to the housing by manual operation in order to establish an operative connection in between the pusher device and a piston of a syringe received on the receptacle.

For this, a releasable brake mechanism is provided. The brake mechanism, in a brake state, serves to rotationally fix the second drive element. Dependent on where the second drive element is arranged, in the brake state the second drive element is rotationally fixed by means of the brake mechanism with respect to the housing of the infusion device (in case the second drive element is arranged on the housing and the first drive element is arranged on the pusher device) or with respect to the pusher device (in case the second drive element is arranged on the pusher device and the first drive element is arranged on the housing of the infusion device).

In the brake state, hence, a force flow is established in between the pusher device and the housing of the infusion device such that, by means of the electric drive, the pusher device may be moved along the pushing direction with respect to the housing. The force flow however may be disrupted by transferring the brake mechanism to a release state in which the rotational fixation of the second drive element is released such that the second drive element may be rotated with respect to the housing of the infusion device (in case the second drive element is arranged on the housing and the first drive element is arranged on the pusher device) or with respect to the pusher device (in case the second drive element is arranged on the pusher device and the first drive element is arranged on the housing of the infusion device). By releasing the rotational fixation of the second drive element, hence, a rotation of the second drive element with respect to the first drive element becomes possible while the first drive element is at standstill. When the force flow between the pusher device and the housing of the infusion device hence is disrupted, the pusher device may be axially moved with respect to the housing along the pushing direction, the second drive element in this case being freely rotatable with respect to the first drive element and hence not hindering a translational movement of the pusher device.

The spindle and the spindle nut are in threaded engagement with one another. The threaded engagement herein is not self-locking. Hence, the outer threading of the spindle and the inner threading of the spindle nut comprise an inclination at such an angle that the second drive element is rotationally moved when acting onto the second drive element for translationally moving the second drive element with respect to the first drive element.

Because a spindle drive is used to drive the pusher device, a drive mechanism of simple construction becomes possible. In that a clutching assembly of complicated structural built is avoided a particularly reliable operation may be achieved, avoiding for example an undesired unclutching and allowing for an improved efficiency and a reduced wear and tear.

In one embodiment, the brake mechanism is configured to, in the brake state, operatively connect the second drive element to the pusher device such that the second drive element is rotationally fixed with respect to the pusher device. The brake mechanism is further configured to, in the release state, operatively disconnect the second drive element from the pusher device such that the second drive element is rotatable with respect to the pusher device. In this embodiment, the second drive element, formed by the spindle or the spindle nut, is arranged on the pusher device. The first drive element, formed by the other of the spindle and the spindle nut, is arranged on the housing of the infusion device and is driven by the electric drive. In the brake state the second drive element is held fixed with respect to the pusher device. In the release state, the second drive element is released from the pusher device in that it is rotatable with respect to the pusher device, hence allowing a translational movement of the second drive element with respect to the first drive element and hence of the pusher device with respect to the housing.

In one embodiment, the second drive element, in the brake state of the brake mechanism, is rotationally and translationally, along the pushing direction, fixed to the pusher device such that a rotational movement of the first drive element causes a translational movement of the pusher device along the pushing direction. In the release state, in turn, the pusher device is translationally movable with respect to the first drive element while the first drive element is at standstill. In the release state the second drive element is rotatable with respect to the pusher device. Hence by pushing on the pusher device the second drive element may rotate with respect to the first drive element, while the first drive element is at standstill, such that the drive mechanism does not hinder an axial, translational movement of the pusher device with respect to the housing of the infusion device.

In one embodiment, the pusher device comprises a pusher housing. The brake mechanism herein acts in between the pusher housing and the second drive element. In particular, the brake mechanism is configured to operatively connect, in the brake state, the second drive element to the pusher housing and to operatively release, in the release state, the second drive element from the pusher housing such that the second drive element may freely rotate with respect to the pusher housing. In the brake state a force flow in between the electric drive on the housing of the infusion device and the pusher device is established such that by driving the first drive element the pusher device may be translationally moved along the pushing direction with respect to the housing. By releasing the brake mechanism, a manual movement of the pusher device independent of the drive mechanism becomes possible, in particular in order to establish a connection of the pusher device to the piston of the syringe, or to release the connection of the pusher device from the piston of the syringe.

The drive mechanism, in one embodiment, comprises a self-locking gearing connecting the electric drive to the first drive element. By means of the self-locking gearing the first drive element, formed by the spindle or the spindle nut, is held rotationally fixed when the electric drive is not energized. When the electric drive is not energized, hence, the first drive element is at standstill and is held in position, wherein the second drive element may be moved with respect to the first drive element when the brake mechanism is in the release state, but otherwise is not movable when the brake mechanism is in the brake state.

The self-locking gearing may for example be formed by a worm gear. The worm gear may for example comprise a drive worm arranged on a motor shaft of the electric drive and engaging, with a worm thread, with a toothing of a drive wheel which is connected to the first drive element, formed by the spindle or the spindle nut.

The brake mechanism may be formed by any brake which is suitable to provide for a rotational fixation of the second drive element. The brake may be formed by a mechanic brake, a magnetic brake, a magnetorheological brake or by any other brake suitable to provide for a rotational fixation of the second drive element.

The brake mechanism comprises a first brake element which is configured to interact, in the brake state, with the second drive element. The first brake element may be movable for transferring the brake mechanism between the brake state and the release state. The first brake element may be approachable towards the second drive element along a radial direction or along an axial direction (with respect to the pushing direction along which the spindle extends and along which the spindle nut and the spindle are translationally movable with respect to one another).

The first brake element may be arranged on the housing of the infusion device (in case the second drive element is arranged on the housing) or on the pusher device (in case the second drive element is arranged on the pusher device).

In one embodiment, the first brake element is arranged on and movable with respect to the pusher device and is tensioned by a spring element with respect to the pusher device towards a braking position associated with the brake state of the brake mechanism. The spring element, for example in the shape of a compression spring, for example acts in between the first brake element and a pusher housing of the pusher device. By means of the spring element the first brake element is tensioned towards an interaction with the second drive element such that, without an actuation of the first brake element, the brake mechanism assumes the brake state and the second drive element is rotationally fixed.

In one embodiment, the second drive element comprises a second brake element which, in the brake state of the brake mechanism, is in abutment with the first brake element. The brake mechanism, in this embodiment, implements a mechanic brake. The first brake element and the second brake element, in the brake state of the brake mechanism, mechanically interact with one another in that the first brake element and the second brake element are in abutment with one another. The second brake element is fixedly arranged on the second drive element, such that in the brake state the second brake element and with it the second drive element are held rotationally fixed by means of the first brake element.

In order to transfer the brake mechanism to the release state, the first brake element is moved with respect to the second brake element such that the abutment of the first brake element and the second brake element is overcome and the second brake element may rotationally move with respect to the first brake element. In the release state, hence, the brake mechanism does not cause a braking action on the second drive element.

The drive mechanism comprises an actuating arrangement for actuating the brake mechanism to transfer the brake mechanism between the brake state and the release state. The actuating arrangement may in particular be actuatable by a user. By acting onto the actuating arrangement, the brake mechanism is transferred, in particular, from the brake state to the release state such that a manual movement of the pusher device with respect to the housing becomes possible without hindrance by the drive mechanism.

In a non-actuated state of the actuating arrangement the brake mechanism is in the brake state. The actuating arrangement herein is configured to move the brake mechanism from the brake state to the release state upon user actuation of the actuating arrangement. By actuating the actuating arrangement, in particular, it may be acted onto a brake element of the brake mechanism in order to release a braking action of the brake element, such that the rotational fixation of the second drive element is overcome and the second drive element becomes rotatable with respect to the first drive element.

The actuating arrangement comprises a first actuating element which is user actuatable for actuating the brake mechanism. The first actuating element may for example be formed by a lever element. A user may act onto the first actuating element in order to actuate the actuating arrangement such that the brake mechanism is transferred between the brake state and the release state.

In case the brake mechanism is arranged on the pusher device, the actuating arrangement likewise is arranged on the pusher device, and the first actuating element is movably arranged on the pusher device. A user may hence act onto the first actuating element on the pusher device, for example by pushing on the first actuating element, in order to in this way actuate the brake mechanism in particular to allow for a manual translational movement of the pusher device with respect to the housing.

The actuating arrangement comprises a second actuating element operatively connected to the brake mechanism and being movable by the first actuating element. The second actuating element may be formed by (another) lever element which in a first state is movable together with the first actuating element and, in a second state, may be independently movable from the first actuating element. By using two actuating elements, for example a so-called anti-bolus mechanism may be provided which prevents the administration of an undesired bolus by manual movement of the pusher device when establishing a connection between the pusher device and a piston of a syringe received on the receptacle when setting up operation of the infusion device.

The actuating arrangement comprises a coupling device for coupling the first actuating element to the second actuating element such that, in a coupling state, the second actuating element is moved together with the first actuating element when actuating the first actuating element. The coupling device is switchable to a non-coupling state in which the second actuating element is movable independently of the first actuating element. The coupling device in particular shall establish a connection between the first actuating element and the second actuating element when the actuating arrangement is actuated in order to manually move the pusher device to establish a connection of the pusher device with a piston of a syringe received on the receptacle. For the manual movement the brake mechanism shall be released. For this a user acts onto the first actuating element and causes the second actuating element to act onto the brake mechanism in order to force the brake mechanism into the release state. Once the pusher device comes into contact with the piston, the coupling device is transferred to the non-coupling state, such that a force action of the second actuating element on the brake mechanism is released and the brake mechanism moves back to the brake state. Upon establishing a contact between the pusher device and the piston, hence, a further movement of the pusher device is prevented and hence an undesired bolus by manual movement of the piston is avoided.

For causing the coupling device to transition from the coupling state to the non-coupling state, the pusher device comprises a detection device for detecting a contact between the pusher device and a piston of a syringe received on the receptacle. Once a contact between the pusher device and the piston is detected by the detection device, the coupling device is caused to switch from the coupling state to the non-coupling state. When the coupling device is switched to the non-coupling state, the second actuating element is able to move with respect to the first actuating element, such that a user force caused on the first actuating element no longer holds the second actuating element in place, but rather the second actuating element may move together with the brake mechanism for example under a tensioning force acting onto a brake element towards the brake state. Once a contact between the pusher device and the piston is detected, thus, the brake mechanism automatically moves back into its brake state, such that a further movement of the pusher device is blocked and a manual movement of the piston, potentially causing an undesired bolus, is prevented.

In one embodiment, the coupling device is configured to, in the coupling state, magnetically connect the first actuating element to the second actuating element. The coupling device for example may comprise an electromagnet which is energized when the first actuating element is actuated by a user and which is de-energized when a contact between the pusher device and the piston is detected. In another embodiment, the coupling device may comprise a permanent magnet and an electromagnet. In this embodiment, the coupling device may, in a default state, establish a coupling between the first actuating element and the second actuating element by means of the permanent magnet. When the coupling shall be released, the electromagnet may be energized to counteract the magnetic force of the permanent magnet, such that magnetic attraction forces between the first actuating element and the second actuating element may be substantially cancelled, hence allowing for a movement of the second actuating element with respect to the first actuating element.

The detection device may be a switch device or another contact sensor, which for example together with a processor causes an energization or de-energization of the coupling device.

In another embodiment, the coupling device may be established as a mechanical device, for example by using a movable pin or the like for establishing a coupling between the first actuating element and the second actuating element. The detection device in this embodiment for example may be a mechanical linking mechanism which is actuated upon contact between the pusher device and the piston for releasing the connection between the first actuating element and the second actuating element.

In other embodiments, the actuating arrangement may have another design. For example, the actuating mechanism may comprise a single actuating element which is actuatable by a user and which directly acts onto the brake mechanism. An anti-bolus mechanism in principal may also be dispensable.

In one embodiment, an energy harvesting technique may be employed when actuating the actuating arrangement. Hence, a mechanical actuation of the actuating arrangement may be used to generate electrical energy, which may for example be fed to an electrical energy storage usable for operation of the anti-bolus mechanism.

One or multiple actuating elements of the actuating arrangement may be formed by lever elements. In another embodiment, one or multiple actuating elements may be implemented by slide or push elements.

In one embodiment, a bidirectional bistable actuator may be used as an actuating arrangement. An actuator of this kind may directly act onto the brake mechanism.

The idea underlying the invention shall subsequently be described in more detail with respect to the embodiments shown in the figures. Herein:
- Fig. 1: shows a view of an embodiment of an infusion device in the shape of a syringe pump;
- Fig. 2: shows a schematic drawing of an embodiment of a drive mechanism of an infusion device;
- Fig. 3: shows a schematic drawing of a gearing for connecting an electric drive to a first drive element;
- Fig. 4: shows the arrangement of Fig. 2, at the start of a user actuation of an actuating arrangement for releasing a brake mechanism;
- Fig. 5: shows the arrangement of Fig. 4, during user actuation;
- Fig. 6: shows the arrangement of Fig. 5, during further user actuation;
- Fig. 7: shows the arrangement of Fig. 6, during further user actuation for translationally moving the pusher device to establish a connection of the pusher device with a piston of a syringe received on a receptacle of the infusion device;
- Fig. 8: shows the arrangement of Fig. 7, upon establishment of a contact between the pusher device and the piston;
- Fig. 9: shows the arrangement of Fig. 8, during a transitioning of the brake mechanism from a release state to a brake state upon the establishment of a contact between the pusher device and the piston;
- Fig. 10: shows the arrangement of Fig. 9, while releasing the user actuation; and
- Fig. 11: shows the arrangement of Fig. 10, in a state in which the pusher device is operatively connected to the piston of the syringe.

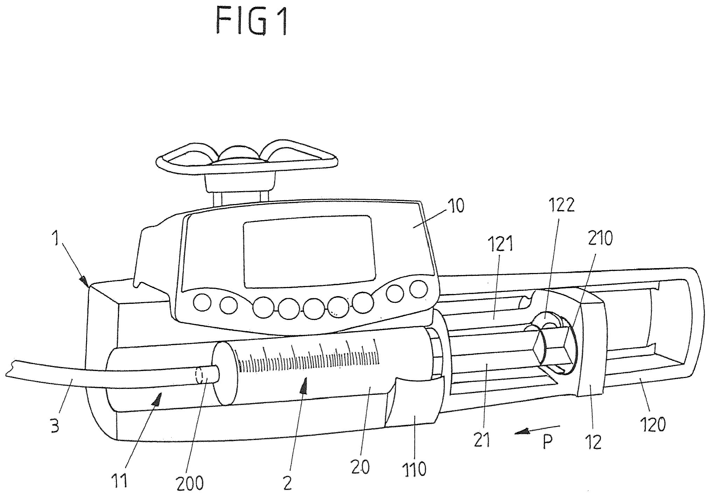

Fig. 1 shows an embodiment of an infusion device 1 in the shape of a syringe pump having a housing 10 and a receptacle 11 arranged on the housing 10 to receive a syringe 2 therein.

The syringe 2 comprises a cylindrical tube 20 which, when installing the syringe 2 on the infusion device 1, contains a medical liquid, for example a medication or a solution for the parenteral feeding, to be infused to a patient. The cylindrical tube 20 is connected, via a connector 200, to an infusion line 3 which may extend from the syringe 2 towards a patient for infusing the medical liquid to the patient.

For installing the syringe 2 on the receptacle 11 of the infusion device 1, the cylindrical tube 20 of the syringe 2 is placed in the receptacle 11 and is mechanically connected to the housing 10 by means of a fixation device 110. By means of the fixation device 110, for example configured by a releasable clamp element, the cylindrical tube 20 is secured within the receptacle 11 such that the cylindrical tube 20 is held in position on the receptacle 11.

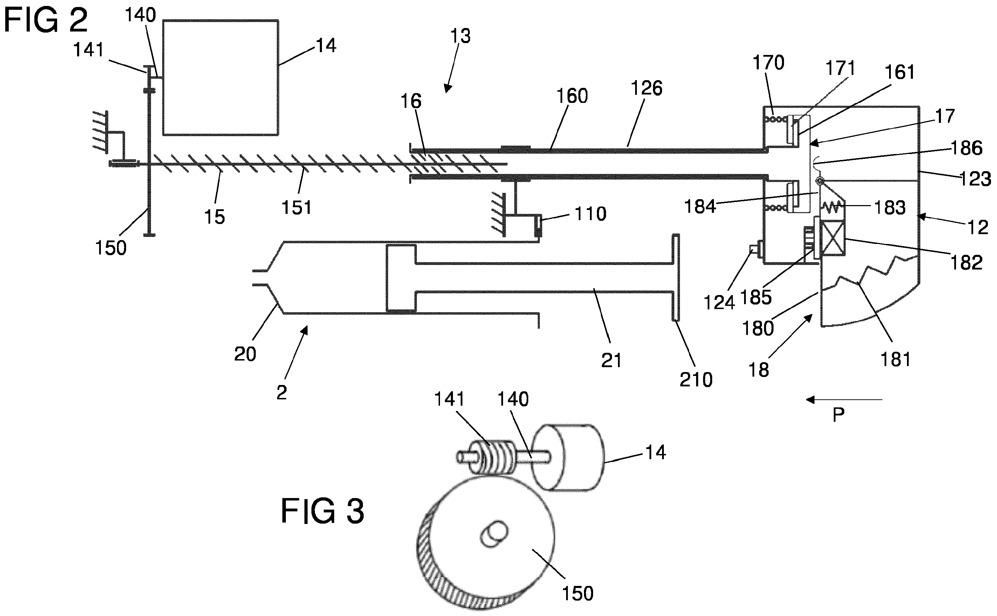

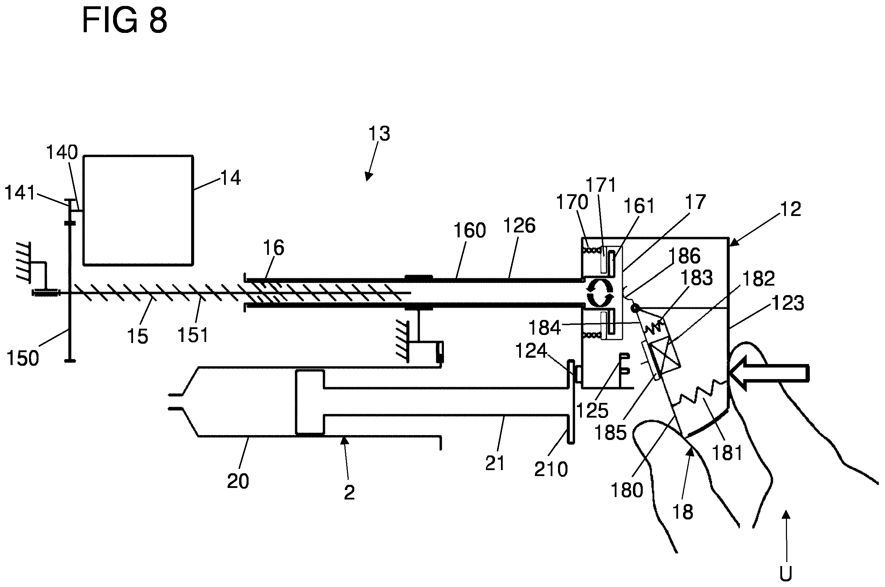

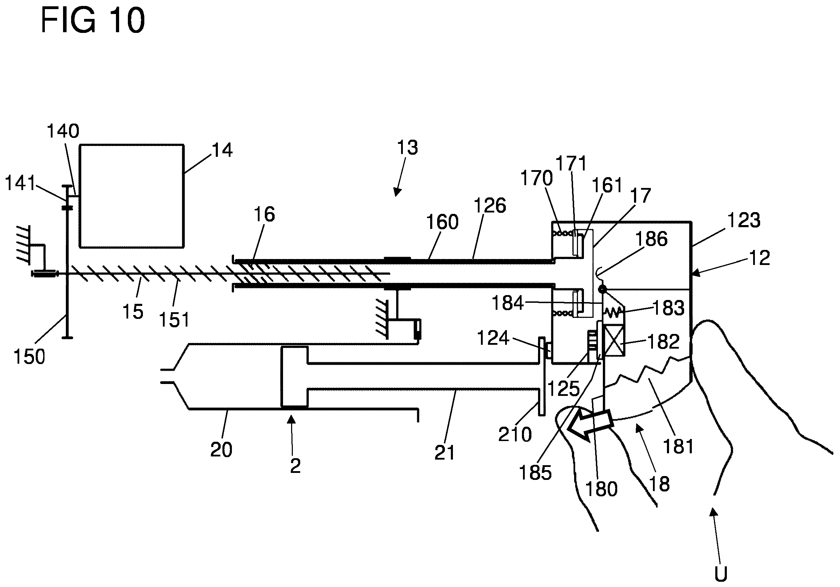

The syringe 2 comprises a piston 21 which, for delivering medical fluid contained in the cylindrical tube 20, can be pushed into the cylindrical tube 20 in a pushing direction P. For this, the infusion device 1 comprises a pusher device 12 movably arranged within a guide device 120 and connected to a drive mechanism (which subsequently shall be described with relation to Figs. 2 to 11) via a connecting rod 121.

For operating the infusion device 1, the syringe 2 is installed on the infusion device 1 and the pusher device 12 is (manually) moved towards a piston head 210 of the piston 21 until the pusher device 12 comes into abutment with the piston head 210. For performing an infusion process the pusher device 12 is then electromechanically moved in the pushing direction P to move the piston 21 into the cylindrical tube 20 for delivering the medical fluid contained in the cylindrical tube 20 via the infusion line 3 towards the patient at a desired infusion rate.

The pusher device 12 is driven by a drive mechanism 13, which, according to one embodiment, is schematically illustrated in Figs. 2 to 11.

The drive mechanism 13 comprises an electric drive 14, a first drive element 15 formed by a spindle, a second drive element 16 formed by a spindle nut, a brake mechanism 17 and an actuating arrangement 18 formed by an arrangement of pivotable levers. In the shown embodiment, the electric drive 14 is arranged stationary within the housing 10 of the infusion device 1. The spindle 15 is operatively connected to the electric drive 14 and is rotatable within the housing 10 of the infusion device 1. The spindle nut 16 is associated with the pusher device 12 and serves to establish a connection of the pusher device 12 to the drive mechanism 13 to move, during regular operation, the pusher device 12 with respect to the housing 10 along the pushing direction P.

The electric drive 14, formed by an electric motor, comprises a motor shaft 140 on which an output element 141 is arranged, which in the embodiment of Fig. 3 is formed by a worm gear. The output element 141 engages with an input element 150 connected to the spindle 15, the output element 141 and the input element 150 together forming a self-locking gearing for establishing an operative connection in between the electric drive 14 and the spindle 15.

The spindle 15, on its outer circumference, carries an outer threading 151 by means of which a threaded engagement with the spindle nut 16 is established. The spindle nut 16 forms an inner threading which engages with the outer threading 151 of the spindle 15, such that a rotation of the spindle 15 causes an axial, translational movement of the spindle nut 16 along the pushing direction P with respect to the spindle 15.

The spindle nut 16 is connected to a shaft 160 which extends within a longitudinal portion 126 connected to the pusher device 12. The spindle nut 16 with the shaft 160 is rotatably mounted within the portion 126 of the pusher device 12, such that the spindle nut 16 (in principle) is rotatable with respect to the pusher device 12.

A brake mechanism 17 serves, during regular operation, to establish an operational connection in between the spindle nut 16 and the pusher device 12. The brake mechanism 17 in particular, during regular operation, establishes a rotationally fixed connection of the spindle nut 16 to a pusher housing 123 of the pusher device 12, such that the spindle nut 16 may not be rotated with respect to the pusher device 12, but is held in place both rotationally and translationally with respect to the pusher device 12.

In the shown embodiment, the brake mechanism 17 comprises a brake element 171 having a disc shape, which in a brake state is in abutment with a disc-shaped brake element 161 formed at an end of the shaft 160 of the spindle nut 16. The brake element 171 is tensioned with respect to the pusher housing 123 of the pusher device 12 by means of a compression spring 170, such that in a non-actuated state the brake mechanism 17 establishes a connection in between the spindle nut 16 and the pusher housing 123 of the pusher device 12.

In the shown embodiment the brake mechanism 17 acts as a mechanical brake. It shall be noted that in principle other brake mechanisms may be employed, for example mechanical brakes of other construction, magnetic brakes or for example a so-called magnetorheological brake.

When the brake mechanism 17 is not actuated, it assumes its brake state, as is shown in Fig. 2. However, the brake mechanism 17 may be actuated in order to release the spindle nut 16, wherein in a release state the brake mechanism 17, with its brake element 171, assumes such a position that the spindle nut 16 may rotate with respect to the pusher housing 123.

For actuating the brake mechanism 17 to transfer the brake mechanism 17 from the brake state to the release state, an actuating arrangement 18 is provided on the pusher device 12, which is user actuatable and allows to act onto the brake element 171 in order to disengage the brake element 171 from the brake element 161 of the spindle nut 16.

The actuating arrangement 18 comprises a first actuating element 180 and a second actuating element 184, which interact for acting onto the brake mechanism 17. The first actuating element 180 herein is user actuatable in that a user may press onto the first actuating element 180. The second actuating element 184, during actuation, is moved together with the first actuating element 180 such that an actuation force is transferred onto the brake mechanism 17 in order to displace the brake element 171 against the tensioning force of the compression spring 170.

In the shown embodiment, both actuating elements 180, 184 are formed by lever elements which are pivotable with respect to the pusher housing 123 of the pusher device 12. It shall be noted that, in principle, other actuating elements, such as movable push or slide elements, may be used instead of lever elements.

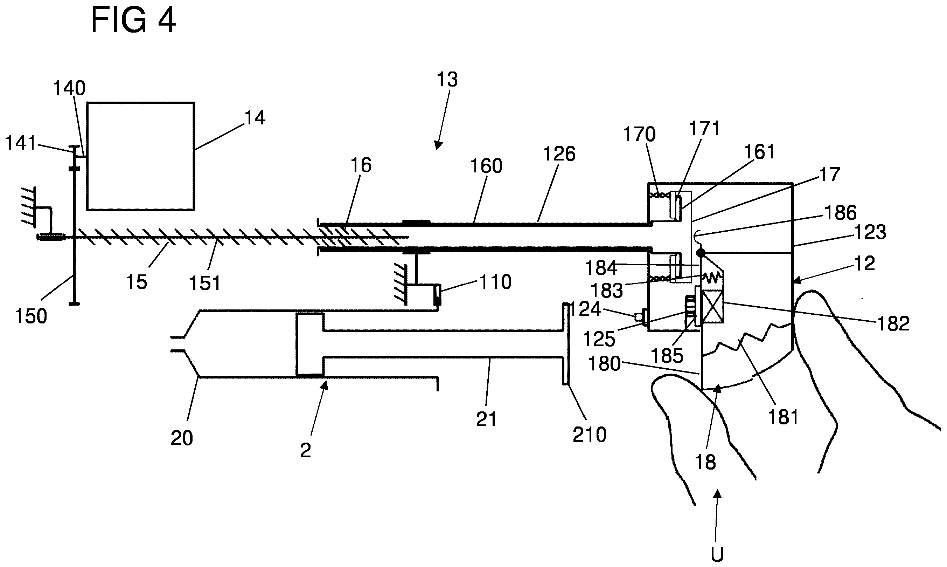

Referring now to Fig. 4, when the electric drive 14 is not energized, the spindle 15 is held in place by means of the self-locking gearing formed by the output element 141 and the input element 150. The self-locking effect may be achieved, for instance, thanks to the mechanical irreversibility of a worm gear or by any other non-reversible gear drive. When a user U wishes to manually move the pusher device 12 in order to establish a connection of the pusher device 12 to a piston 21 of a syringe 2 received on the receptacle 11, the user U actuates the actuating arrangement 18 by pressing onto the actuating element 180, as it is visible in the transition from Fig. 4 to Fig. 5. The actuating element 180 herein is moved against a compressional force of a spring element 181.

The first actuating element 180 is tensioned with respect to the second actuating element 184 by a spring element 183 formed by a traction spring. When the actuating element 180 hence is actuated by the user U, due to the tractional force of the spring element 183 the actuating element 184 is moved together with the actuating element 180, thus removing the actuating element 184 from a sensor device 125, for example in the shape of an optical sensor. The sensor device 125 detects the movement of the actuating element 184 and outputs a signal, causing a coupling device 182 formed by an electromagnet to be energized such that a magnetic attraction force is produced between the coupling device 182 fixedly arranged on the actuating element 180 and a counter element 185 fixedly arranged on the actuating element 184, thus establishing a connection in between the actuating elements 180, 184.

This configuration serves the purpose to detect an intention of a user to actuate the brake mechanism 17 and to trigger, as a consequence, the energization of the electromagnet. Therefore, the electromagnet is only supplied with energy when the user intends to manually move the pusher device 12.

In another embodiment, the coupling device 182 formed by the electromagnet may be continuously energized, hence alleviating the need for the spring element 183 and the sensor device 125. This may reduce the mechanical complexity but comes with the drawback of a higher electrical consumption.

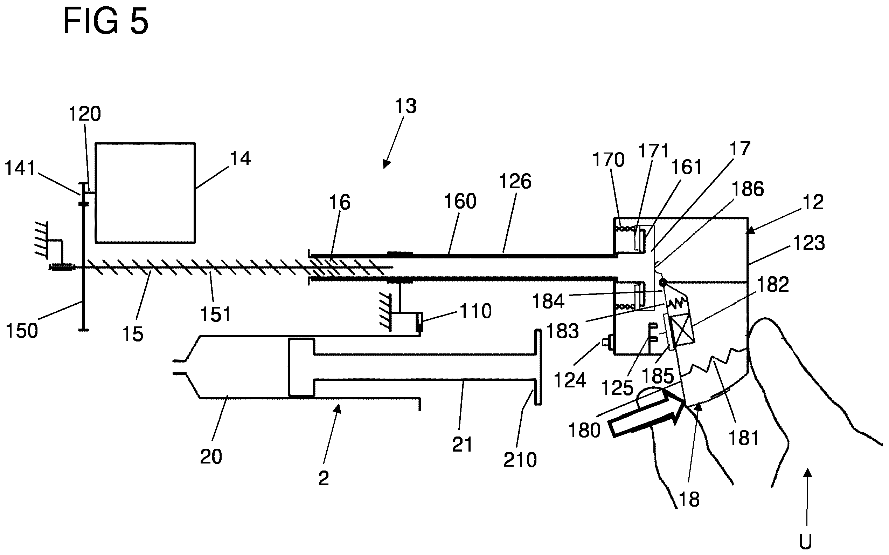

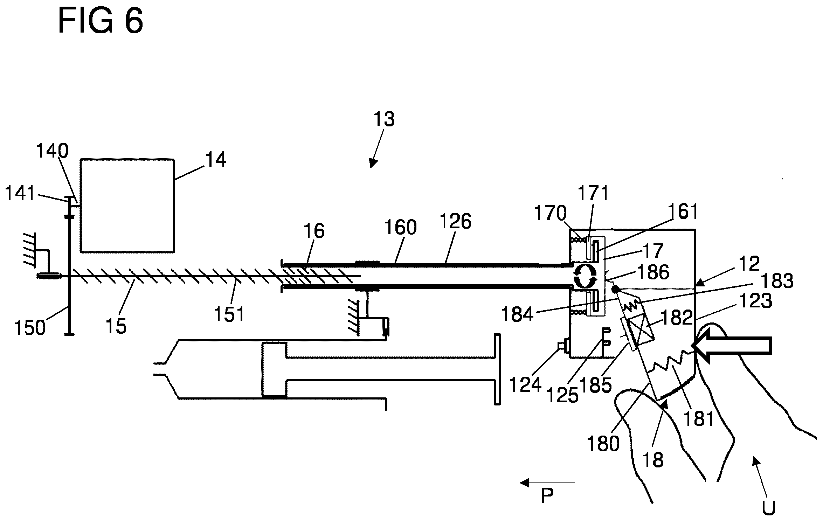

When further moving the actuating element 180, hence, the actuating element 184 is moved together with the actuating element 180, as visible in the transition between Fig. 5 to Fig. 6. The actuating element 184, with an end opposite the user-actuated portion of the actuating element 180, acts onto the brake element 171 of the brake mechanism 17 and pushes the brake element 171 out of abutment from the brake element 161 of the spindle nut 16, against the compressional tensioning force of the spring element 170. The spindle nut 16 hence is released, such that the spindle nut 16 is free to rotate with respect to the pusher housing 123 of the pusher device 12, as visible from Fig. 6.

The threaded engagement of the spindle nut 16 with the spindle 15 is not self-locking, such that a pushing on the spindle nut 16 along the pushing direction P is transferred into a rotational movement of the spindle nut 16 with respect to the spindle 15, without a locking counteracting the rotation. The non-self-locking effect is ensured thanks to a specific design of the spindle nut 16 and its nut threads as stated in the known art. The user U hence may move the pusher device 12 in the pushing direction P, as visible from the transition from Fig. 6 to Fig. 7, such that the pusher device 12 may be approached towards the piston head 210 of the piston 21 of the syringe 2 received on the receptacle 11 of the infusion device 1.

During the translational movement of the pusher device 12, the spindle nut 16 is rotated with respect to the pusher device 12, while the brake mechanism 17 due to the actuation of the actuating arrangement 18 is in its release state.

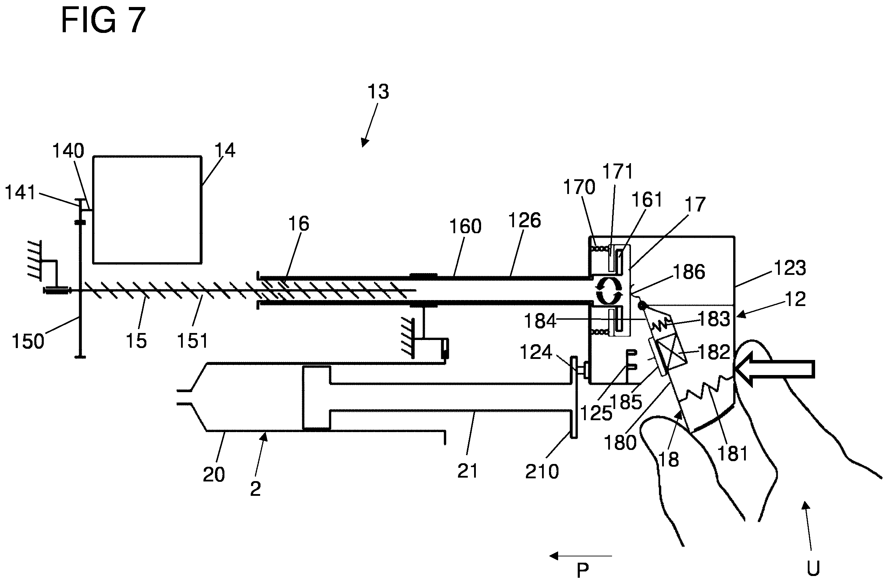

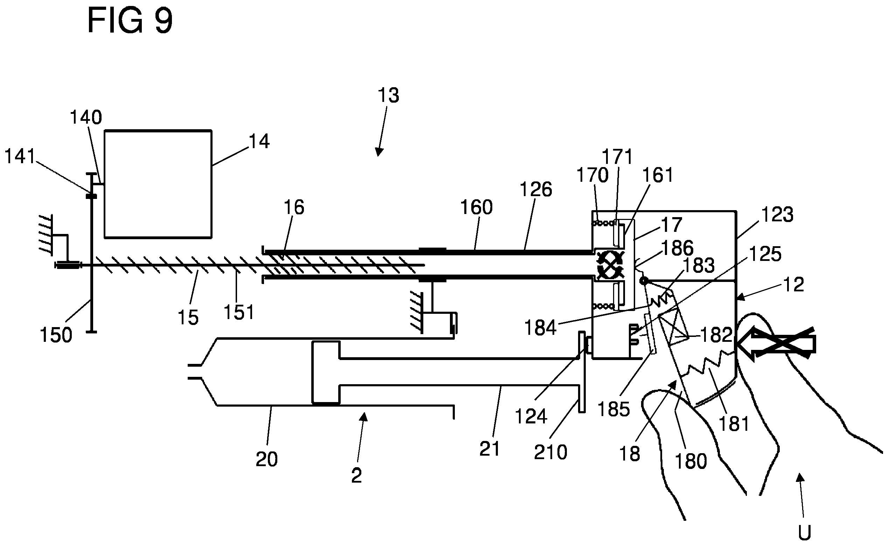

When the pusher device 12 comes into contact with the piston head 210, a detection device 124 on the pusher housing 123 is actuated, as shown in the transition of Fig. 7 to Fig. 8. The detection device 124 may for example be formed by a switch, which generates a signal causing the coupling device 182 to be de-energized such that the coupling between the actuating elements 180, 184 is released. When the coupling is released, the second actuating element 184 is free to move with respect to the first actuating element 180, such that due to the compressional force exerted onto the brake element 171 by means of the spring element 170 the brake element 171 snaps back to its brake position and abuts with the brake element 161 of the spindle nut 16, the actuating element 184 being pushed along and being moved back towards its initial position, as visible from Fig. 9.

The compression spring 170 and the traction spring 183 are dimensioned such that, in the configuration of Fig. 7, the torque created by the compression spring is much higher than the torque created by the traction spring 183, in order for the second actuating element 184 to be pushed back towards its initial position.

Once contact between the pusher device 12 and the piston 21 is established, the brake mechanism 17 hence resets back to its brake state, such that a further rotational movement of the spindle nut 16 with respect to the pusher housing 123 is prevented and the pusher device 12, due to the fixation of the spindle nut 16, is held in place. A pushing action onto the pusher device 12 hence may not cause a manual movement of the piston 21, hence preventing an undesired bolus during the installation procedure.

When the user U now releases the actuating element 180, the actuating element 180 moves back into its starting position due to the compressional force exerted by the spring element 181, as shown in the transition from Fig. 9 to Fig. 10.

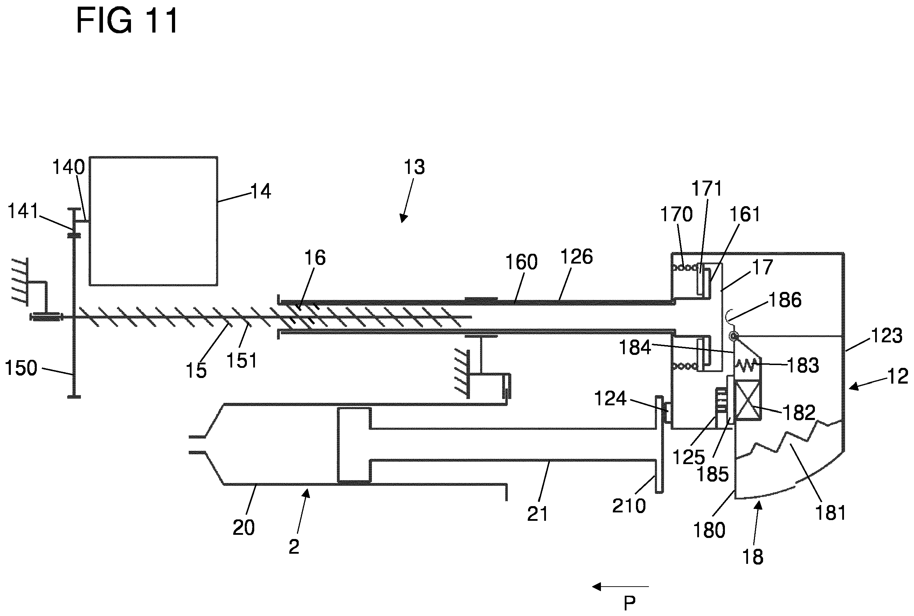

When actuation has ended, as visible in Fig. 11, the brake mechanism 17 is in its brake state. The pusher device 12 is in abutment with the piston 21 such that an operative connection in between the pusher device 12 and the piston 21 is established, which may further be secured for example using a fastening element 122 formed by a so-called anti-siphon arm, as shown in Fig. 1, in order to allow for a back-and-forth movement of the piston 21 by moving the pusher device 12.

Once the operative connection in between the pusher device 12 and the piston 21 is established, the pusher device 12 may regularly be moved by driving the spindle 15 using the electric drive 14. In particular, the spindle 15 is rotated by the electric drive 14, by which the spindle nut 16 is translationally moved along the pushing direction P and, together with the spindle nut 16, the pusher device 12 is moved in order to act onto the piston 21.

When a user U wishes to again release the pusher device 12 from the piston 21, the user U may again actuate the actuating arrangement 18 by pressing the actuating element 180, similarly as shown in Fig. 4. By releasing the brake mechanism 17, the pusher device 12 may be separated from the piston 21 by manually moving the pusher device 12 opposite to the pushing direction P. The syringe 2 may hence be removed from the receptacle 11 and may for example be replaced by another syringe 2 in order to continue infusion.

The idea underlying the invention is not limited to the embodiments described above. The invention is defined by the claims.

The actuating arrangement may comprise one or multiple actuating elements. In particular, if an anti-bolus mechanism is deemed dispensable, a single actuating element may be used which is user actuatable and which directly acts onto the brake mechanism in order to actuate the brake mechanism.

Different constructions of an actuating arrangement are conceivable. An actuating arrangement may use one or multiple lever elements or other elements, such as push or slide elements.

Also, different constructions of the brake mechanism are conceivable. A brake mechanism may act as a mechanical, magnetic or a magnetorheological brake. In a mechanic brake, brake elements may be approachable with one another axially along the pushing direction or radially with respect to a rotational axis.

It is also possible to use an electric brake activated by a push button triggering the electric supply of the electric brake. This solution may allow to get rid of the springs and levers of the above-described solution.

In the shown embodiment, the spindle nut is arranged on the pusher device. In other embodiments, a spindle may be arranged on the pusher device, whereas a spindle nut is arranged on the housing of the infusion device.

In the shown embodiment, the spindle is in operative connection with the electric drive and is driven by the electric drive for performing an infusion operation. In other, kinematically reversed embodiments, the spindle nut may be in operative connection with and driven by the electric drive, wherein in this case the brake mechanism is associated with the spindle for braking the spindle.

By using a spindle drive, a simple construction may be employed, which is reliable and efficient in operation and allows for a low wear and tear.

### List of reference numerals

- 1: Infusion device
- 10: Housing
- 11: Receptacle
- 110: Fixation device
- 12: Pusher device
- 120: Guide device
- 121: Connecting rod
- 122: Fastening element (anti-siphon arm)
- 123: Pusher housing
- 124: Detection device
- 125: Sensor device (optical sensor)
- 126: Housing portion
- 13: Drive mechanism
- 14: Electric drive
- 140: Motor shaft
- 141: Output element
- 15: Drive element (spindle)
- 150: Input element
- 151: Outer threading
- 16: Drive element (spindle nut)
- 160: Shaft
- 161: Brake element
- 17: Brake mechanism
- 170: Spring element (compression spring)
- 171: Brake element
- 18: Actuating arrangement
- 180: Actuating element (lever element)
- 181: Spring element (compression spring)
- 182: Coupling device (electromagnet device)
- 183: Spring element (traction spring)
- 184: Actuating element (lever element)
- 185: Counter element
- 186: End
- 2: Syringe
- 20: Cylinder tube
- 200: Connector
- 21: Piston
- 210: Piston head
- 3: Infusion line
- P: Pushing direction
- U: User

## Claims

1. Infusion device (1) for administering a medical fluid to a patient, comprising:
a housing (10) having a receptacle (11) for receiving a syringe (2),
a pusher device (12) which is movable along a pushing direction (P) for acting onto a piston (21) of a syringe (2) received on the receptacle (11), and
a drive mechanism (13) for driving the pusher device (12), the drive mechanism (13) comprising
a first drive element (15),
a second drive element (16) being in threaded engagement with the first drive element (15), the second drive element (16) being translationally movable with respect to the first drive element (15) by rotating the first drive element (15), and
an electric drive (14) for rotating the first drive element (15),
**wherein** one of the first drive element (15) and the second drive element (16) is a spindle having an outer threading (151) and the other of the first drive element (15) and the second drive element (16) is a spindle nut being in threaded engagement with the outer threading (151) of the spindle,
wherein the drive mechanism (13) comprises a brake mechanism (17) for rotationally fixing, in a brake state, the second drive element (16) such that a rotation of the first drive element (15) causes a translational movement of the second drive element (16), and for rotationally releasing, in a release state, the second drive element (16) to allow a rotation of the second drive element (16) with respect to the first drive element (15) while the first drive element (15) is at standstill,
wherein the brake mechanism (17) comprises a first brake element (171) which is configured to interact, in the brake state, with the second drive element (16), and
wherein the first brake element (171) is arranged on and movable with respect to the pusher device (12) and is tensioned by a spring element (170) with respect to the pusher device (12) towards a braking position associated with the brake state of the brake mechanism (17),
wherein the drive mechanism (13) comprises an actuating arrangement (18) for actuating the brake mechanism (17) to transfer the brake mechanism (17) between the brake state and the release state,
wherein in a non-actuated state of the actuating arrangement (18) the brake mechanism (17) is in the brake state, wherein the actuating arrangement (18) is configured to move the brake mechanism (17) from the brake state to the release state upon user actuation of the actuating arrangement (18),
**characterized in that** the actuating arrangement (18) comprises a first actuating element (180) which is user actuatable for actuating the brake mechanism (17) and the actuating arrangement (18) comprises a second actuating element (184) operatively connected to the brake mechanism (17) and being movable by the first actuating element (180), and the actuating arrangement (18) comprises a coupling device (182) for coupling the first actuating element (180) to the second actuating element (184) such that, in a coupling state, the second actuating element (184) is moved together with the first actuating element (180) when actuating the first actuating element (180), wherein the coupling device (182) is switchable to a non-coupling state in which the second actuating element (184) is movable independently of the first actuating element (180),
wherein the pusher device (12) comprises a detection device (124) for detecting a contact between the pusher device (12) and a piston (21) of a syringe (2) received on the receptacle (11), the actuating arrangement (18) being configured such that a contact between the pusher device (12) and the piston (21) detected by the detection device (124) causes the coupling device (182) to be switched from the coupling state to the non-coupling state, the switching of the coupling device (182) from the coupling state to the non-coupling state causing the brake mechanism (17) to transfer from the release state to the brake state.

2. Infusion device (1) according to claim 1, **characterized in that** the brake mechanism (17) is configured to, in the brake state, operatively connect the second drive element (16) to the pusher device (12) such that the second drive element (16) is rotationally fixed with respect to the pusher device (12), wherein the brake mechanism (17) is further configured to, in the release state, operatively disconnect the second drive element (16) from the pusher device (12) such that the second drive element (16) is rotatable with respect to the pusher device (12).

3. Infusion device (1) according to claim 1 or 2, **characterized in that** the second drive element (16), in the brake state of the brake mechanism (17), is rotationally and translationally, along the pushing direction (P), fixed to the pusher device (12) such that a rotational movement of the first drive element (15) causes a translational movement of the pusher device (12) along the pushing direction (P), wherein in the release state the pusher device (12) is translationally movable with respect to the first drive element (15) while the first drive element (15) is at standstill.

4. Infusion device (1) according to one of claims 1 to 3, **characterized in that** the pusher device (12) comprises a pusher housing (123), wherein the brake mechanism (17) is configured to operatively connect, in the brake state, the second drive element (16) to the pusher housing (123) and to operatively release, in the release state, the second drive element (16) from the pusher housing (123).

5. Infusion device (1) according to one of the preceding claims, **characterized in that** the drive mechanism (13) comprises a self-locking gearing (141, 150) connecting the electric drive (14) to the first drive element (15).

6. Infusion device (1) according to claim 4 or 5, **characterized in that** the second drive element (16) comprises a second brake element (161) which, in the brake state of the brake mechanism (17), is in abutment with the first brake element (171).

7. Infusion device (1) according to claim 1, **characterized in that** the coupling device (182) is configured to, in the coupling state, magnetically connect the first actuating element (180) to the second actuating element (184).

## Patentansprüche

1. Infusionsvorrichtung (1) zum Verabreichen eines medizinischen Fluids an einen Patienten, umfassend:
ein Gehäuse (10) mit einem Behältnis (11) zum Aufnehmen einer Spritze (2),
eine Schiebervorrichtung (12), die entlang einer Schubrichtung (P) bewegbar ist, um auf einen Kolben (21) einer in dem Behältnis (11) aufgenommenen Spritze (2) einzuwirken, und
einen Antriebsmechanismus (13) zum Antreiben der Schiebervorrichtung (12), wobei der Antriebsmechanismus (13) Folgendes umfasst:
ein erstes Antriebselement (15),
ein zweites Antriebselement (16), das mit dem ersten Antriebselement (15) in Gewindeeingriff steht, wobei das zweite Antriebselement (16) durch Drehen des ersten Antriebselements (15) in Bezug auf das erste Antriebselement (15) translatorisch bewegbar ist, und
einen elektrischen Antrieb (14) zum Drehen des ersten Antriebselements (15),
**wobei** eines von dem ersten Antriebselement (15) und dem zweiten Antriebselement (16) eine Spindel mit einem Außengewinde (151) ist und das andere von dem ersten Antriebselement (15) und dem zweiten Antriebselement (16) eine Spindelmutter ist, die in Gewindeeingriff mit dem Außengewinde (151) der Spindel steht,
wobei der Antriebsmechanismus (13) einen Bremsmechanismus (17) umfasst, um das zweite Antriebselement (16) in einem Bremszustand drehfest zu machen, sodass eine Drehung des ersten Antriebselements (15) eine Translationsbewegung des zweiten Antriebselements (16) bewirkt, und um das zweite Antriebselement (16) in einem Freigabezustand drehbar freizugeben, um eine Drehung des zweiten Antriebselements (16) relativ zum ersten Antriebselement (15) zu ermöglichen, während das erste Antriebselement (15) stillsteht,
wobei der Bremsmechanismus (17) ein erstes Bremselement (171) umfasst, das dazu konfiguriert ist, im Bremszustand mit dem zweiten Antriebselement (16) zusammenzuwirken, und
wobei das erste Bremselement (171) an der Schiebervorrichtung (12) angeordnet und in Bezug auf diese beweglich ist und durch ein Federelement (170) in Bezug auf die Schiebervorrichtung (12) in Richtung einer dem Bremszustand des Bremsmechanismus (17) zugeordneten Bremsposition vorgespannt ist,
wobei der Antriebsmechanismus (13) eine Betätigungsanordnung (18) zum Betätigen des Bremsmechanismus (17) umfasst, damit der Bremsmechanismus (17) zwischen dem Bremszustand und dem Freigabezustand übergeht,
wobei sich der Bremsmechanismus (17) in einem nicht betätigten Zustand der Betätigungsanordnung (18) im Bremszustand befindet, wobei die Betätigungsanordnung (18) dazu konfiguriert ist, den Bremsmechanismus (17) bei Betätigung der Betätigungsanordnung (18) durch den Benutzer vom Bremszustand in den Freigabezustand zu bewegen,
**dadurch gekennzeichnet, dass** die Betätigungsanordnung (18) ein erstes Betätigungselement (180) umfasst, das vom Benutzer zum Betätigen des Bremsmechanismus (17) betätigt werden kann, und die Betätigungsanordnung (18) ein zweites Betätigungselement (184) umfasst, das betriebsmäßig mit dem Bremsmechanismus (17) verbunden ist und durch das erste Betätigungselement (180) bewegt werden kann, und die Betätigungsanordnung (18) eine Kopplungsvorrichtung (182) zum Koppeln des ersten Betätigungselements (180) mit dem zweiten Betätigungselement (184) umfasst, sodass in einem Kopplungszustand das zweite Betätigungselement (184) zusammen mit dem ersten Betätigungselement (180) bewegt wird, wenn das erste Betätigungselement (180) betätigt wird, wobei die Kopplungsvorrichtung (182) in einen Nicht-Kopplungszustand umschaltbar ist, in dem das zweite Betätigungselement (184) unabhängig vom ersten Betätigungselement (180) bewegt werden kann,
wobei die Schiebervorrichtung (12) eine Erkennungsvorrichtung (124) zum Erkennen eines Kontakts zwischen der Schiebervorrichtung (12) und einem Kolben (21) einer im Behältnis (11) aufgenommenen Spritze (2) umfasst, wobei die Betätigungsanordnung (18) so konfiguriert ist, dass ein von der Erkennungsvorrichtung (124) erkannter Kontakt zwischen der Schiebervorrichtung (12) und dem Kolben (21) bewirkt, dass die Kopplungsvorrichtung (182) vom Kopplungszustand in den Nicht-Kopplungszustand umgeschaltet wird, wobei das Umschalten der Kopplungsvorrichtung (182) vom Kopplungszustand in den Nicht-Kopplungszustand bewirkt, dass der Bremsmechanismus (17) vom Freigabezustand in den Bremszustand übergeht.

2. Infusionsvorrichtung (1) nach Anspruch 1, **dadurch gekennzeichnet, dass** der Bremsmechanismus (17) dazu konfiguriert ist, im Bremszustand das zweite Antriebselement (16) mit der Schiebervorrichtung (12) betriebsmäßig zu verbinden, sodass das zweite Antriebselement (16) in Bezug auf die Schiebervorrichtung (12) drehfest ist, wobei der Bremsmechanismus (17) ferner dazu konfiguriert ist, im Freigabezustand das zweite Antriebselement (16) von der Schiebervorrichtung (12) betriebsmäßig zu trennen, sodass das zweite Antriebselement (16) in Bezug auf die Schiebervorrichtung (12) drehbar ist.

3. Infusionsvorrichtung (1) nach Anspruch 1 oder 2, **dadurch gekennzeichnet, dass** das zweite Antriebselement (16) im Bremszustand des Bremsmechanismus (17) drehfest und translatorisch entlang der Schubrichtung (P) an der Schiebervorrichtung (12) fixiert ist, so dass eine Drehbewegung des ersten Antriebselements (15) eine Translationsbewegung der Schiebervorrichtung (12) entlang der Schubrichtung (P) bewirkt, wobei die Schiebervorrichtung (12) im Freigabezustand translatorisch in Bezug auf das erste Antriebselement (15) beweglich ist, während das erste Antriebselement (15) stillsteht.

4. Infusionsvorrichtung (1) nach einem der Ansprüche 1 bis 3, **dadurch gekennzeichnet, dass** die Schiebervorrichtung (12) ein Schiebergehäuse (123) umfasst, wobei der Bremsmechanismus (17) dazu konfiguriert ist, dass er im Bremszustand das zweite Antriebselement (16) betriebsmäßig mit dem Schiebergehäuse (123) verbindet und im Freigabezustand das zweite Antriebselement (16) betriebsmäßig vom Schiebergehäuse (123) freigibt.

5. Infusionsvorrichtung (1) nach einem der vorhergehenden Ansprüche, **dadurch gekennzeichnet, dass** der Antriebsmechanismus (13) ein selbsthemmendes Getriebe (141, 150) umfasst, das den elektrischen Antrieb (14) mit dem ersten Antriebselement (15) verbindet.

6. Infusionsvorrichtung (1) nach Anspruch 4 oder 5, **dadurch gekennzeichnet, dass** das zweite Antriebselement (16) ein zweites Bremselement (161) umfasst, das im Bremszustand des Bremsmechanismus (17) am ersten Bremselement (171) anliegt.

7. Infusionsvorrichtung (1) nach Anspruch 1, **dadurch gekennzeichnet, dass** die Kopplungsvorrichtung (182) dazu konfiguriert ist, im Kopplungszustand das erste Betätigungselement (180) magnetisch mit dem zweiten Betätigungselement (184) zu verbinden.

## Revendications

1. Dispositif de perfusion (1) pour administrer un fluide médical à un patient, comprenant :
un logement (10) ayant un réceptacle (11) pour recevoir une seringue (2),
- un dispositif poussoir (12) qui est mobile le long d'une direction de poussée (P) pour agir sur un piston (21) d'une seringue (2) reçue sur le réceptacle (11), et
un mécanisme d'entraînement (13) pour entraîner le dispositif poussoir (12), le mécanisme d'entraînement (13) comprenant
un premier élément d'entraînement (15),
un second élément d'entraînement (16) étant en prise filetée avec le premier élément d'entraînement (15), le second élément d'entraînement (16) étant mobile en translation par rapport au premier élément d'entraînement (15) par rotation du premier élément d'entraînement (15), et
un entraînement électrique (14) pour entraîner en rotation le premier élément d'entraînement (15),
**dans lequel** l'un parmi le premier élément d'entraînement (15) et le second élément d'entraînement (16) est une broche ayant un filetage externe (151) et l'autre parmi le premier élément d'entraînement (15) et le second élément d'entraînement (16) est un écrou de broche étant en prise filetée avec le filetage externe (151) de la broche,
dans lequel le mécanisme d'entraînement (13) comprend un mécanisme de frein (17) pour immobiliser en rotation, dans un état de frein, le second élément d'entraînement (16) de telle sorte qu'une rotation du premier élément d'entraînement (15) provoque un mouvement de translation du second élément d'entraînement (16), et pour libérer en rotation, dans un état de libération, le second élément d'entraînement (16) pour permettre une rotation du second élément d'entraînement (16) par rapport au premier élément d'entraînement (15) alors que le premier élément d'entraînement (15) est à l'arrêt,
dans lequel le mécanisme de frein (17) comprend un premier élément de frein (171) qui est conçu pour interagir, dans l'état de frein, avec le second élément d'entraînement (16), et
dans lequel le premier élément de frein (171) est agencé sur le dispositif poussoir (12) et est mobile par rapport à celui-ci, et est mis sous tension par un élément ressort (170) par rapport au dispositif poussoir (12) vers une position de freinage associée à l'état de frein du mécanisme de frein (17),
dans lequel le mécanisme d'entraînement (13) comprend un agencement d'actionnement (18) destiné à actionner le mécanisme de frein (17) pour effectuer un transfert du mécanisme de frein (17) entre l'état de frein et l'état de libération,
dans lequel, dans un état non actionné de l'agencement d'actionnement (18), le mécanisme de frein (17) est dans l'état de frein, dans lequel l'agencement d'actionnement (18) est conçu pour entraîner en mouvement le mécanisme de frein (17) de l'état de frein à l'état de libération lors d'un actionnement par l'utilisateur de l'agencement d'actionnement (18),
**caractérisé en ce que** l'agencement d'actionnement (18) comprend un premier élément d'actionnement (180) qui peut être actionné par l'utilisateur pour actionner le mécanisme de frein (17) et l'agencement d'actionnement (18) comprend un second élément d'actionnement (184) relié de manière fonctionnelle au mécanisme de frein (17) et étant mobile par le premier élément d'actionnement (180), et l'agencement d'actionnement (18) comprend un dispositif d'accouplement (182) pour accoupler le premier élément d'actionnement (180) au second élément d'actionnement (184) de telle sorte que, dans un état d'accouplement, le second élément d'actionnement (184) est entraîné en mouvement conjointement avec le premier élément d'actionnement (180) lors de l'actionnement du premier élément d'actionnement (180), dans lequel le dispositif d'accouplement (182) peut être commuté dans un état de non-accouplement dans lequel le second élément d'actionnement (184) est mobile indépendamment du premier élément d'actionnement (180),
dans lequel le dispositif poussoir (12) comprend un dispositif de détection (124) pour détecter un contact entre le dispositif poussoir (12) et un piston (21) d'une seringue (2) reçue sur le réceptacle (11), l'agencement d'actionnement (18) étant conçu de telle sorte qu'un contact entre le dispositif poussoir (12) et le piston (21) détecté par le dispositif de détection (124) provoque la commutation du dispositif d'accouplement (182) de l'état d'accouplement à l'état de non-accouplement, la commutation du dispositif d'accouplement (182) de l'état d'accouplement à l'état de non-accouplement provoquant le transfert du mécanisme de frein (17) de l'état de libération à l'état de frein.

2. Dispositif de perfusion (1) selon la revendication 1, **caractérisé en ce que** le mécanisme de frein (17) est conçu pour, dans l'état de frein, relier de manière fonctionnelle le second élément d'entraînement (16) au dispositif poussoir (12) de telle sorte que le second élément d'entraînement (16) est immobilisé en rotation par rapport au dispositif poussoir (12), dans lequel le mécanisme de frein (17) est en outre conçu pour, dans l'état de libération, séparer de manière fonctionnelle le second élément d'entraînement (16) du dispositif poussoir (12) de telle sorte que le second élément d'entraînement (16) est rotatif par rapport au dispositif poussoir (12).

3. Dispositif de perfusion (1) selon la revendication 1 ou 2, **caractérisé en ce que** le second élément d'entraînement (16), dans l'état de frein du mécanisme de frein (17), est immobilisé en rotation et en translation, le long de la direction de poussée (P), sur le dispositif poussoir (12) de telle sorte qu'un mouvement de rotation du premier élément d'entraînement (15) provoque un mouvement de translation du dispositif poussoir (12) le long de la direction de poussée (P), dans lequel, dans l'état de libération, le dispositif poussoir (12) est mobile en translation par rapport au premier élément d'entraînement (15) tandis que le premier élément d'entraînement (15) est à l'arrêt.

4. Dispositif de perfusion (1) selon l'une des revendications 1 à 3, **caractérisé en ce que** le dispositif poussoir (12) comprend un logement de poussoir (123), dans lequel le mécanisme de frein (17) est conçu pour relier de manière fonctionnelle, dans l'état de frein, le second élément d'entraînement (16) au logement de poussoir (123) et pour libérer de manière fonctionnelle, dans l'état de libération, le second élément d'entraînement (16) du logement de poussoir (123).

5. Dispositif de perfusion (1) selon l'une des revendications précédentes, **caractérisé en ce que** le mécanisme d'entraînement (13) comprend un engrenage autobloquant (141, 150) reliant l'entraînement électrique (14) au premier élément d'entraînement (15).

6. Dispositif de perfusion (1) selon la revendication 4 ou 5, **caractérisé en ce que** le second élément d'entraînement (16) comprend un second élément de frein (161) qui, dans l'état de frein du mécanisme de frein (17), est en butée avec le premier élément de frein (171).

7. Dispositif de perfusion (1) selon la revendication 1, **caractérisé en ce que** le dispositif d'accouplement (182) est conçu pour, dans l'état d'accouplement, relier magnétiquement le premier élément d'actionnement (180) au second élément d'actionnement (184).
